# EUROPÄISCHE PATENTSCHRIFT

(11) **EP 4 358 888 B1**
(45) Veröffentlichungstag und Bekanntmachung des Hinweises auf die Patenterteilung: **29.07.2026**
(21) Anmeldenummer: 23744375.9
(22) Anmeldetag: 07.07.2023
(51) Int. Cl.: A61B 34/00, A61B 34/30, A61B 1/00, A61B 17/00

(54) **MIKROROBOTISCHE EINHEIT ZUR FORTBEWEGUNG UND POSITIONIERUNG IN ORGANISCHEN HOHLRÄUMEN**
MICROROBOTIC UNIT FOR PROPULSION MOVEMENT AND POSITIONING IN ORGANIC CAVITIES
UNITÉ MICROROBOTIQUE À DES FINS DE MOUVEMENT DE PROPULSION ET DE POSITIONNEMENT DANS DES CAVITÉS ORGANIQUES

(30) Priorität: 12.07.2022 DE 102022117389
(43) Veröffentlichungstag der Anmeldung: 01.05.2024
(73) Patentinhaber: Karguth, Andreas, 99869 Tüttleben (DE); Festo SE & Co. KG, 73734 Esslingen (DE)
(72) Erfinder: Karguth, Andreas, 99869 Tüttleben (DE)
(74) Vertreter: Engel, Christoph Klaus
(86) Internationale Anmeldenummer: PCT/EP2023/068941
(87) Internationale Veröffentlichungsnummer: WO 2024/013035

(56) Entgegenhaltungen:
- JP-A- H02 141 657
- US-A- 5 662 587
- US-A1- 2007 179 339
- US-A1- 2020 221 938
- US-A1- 2020 305 796

## Beschreibung

Die vorliegende Erfindung betrifft eine mikrorobotische Einheit zur Fortbewegung und Positionierung in organischen Hohlräumen. Diese Einheit umfasst mindestens ein vorderes Spreizmodul und ein hinteres Spreizmodul, ein Schubmodul, welches zwischen den beiden Spreizmodulen angeordnet und mit diesen verbunden ist, sowie einen Versorgungsanschluss, welcher sich axial am hinteren Spreizmodul anschließt und der Zufuhr mindestens eines, bevorzugt mehrerer Versorgungsmedien dient.

In unterschiedlichen Bereichen der Technik besteht der Bedarf, zur Erkundung und/oder zur Ausführung von Reparaturen oder dergleichen, in schwer zugängliche Hohlräume mit miniaturisierten technischen Einheiten vorzudringen. Ebenso besteht bei medizinischen Anwendungen der Bedarf zum Vordringen bzw. zur Navigation zwischen weichen (nachgiebigen) Gewebestrukturen, insbesondere in den sogenannten Organfalten. Beispielsweise gibt es für solche Aufgaben extern aktuierte Endoskope, mit denen das Innere von Organismen oder technischen Hohlräumen untersucht und Strukturen manipuliert werden können. Es sind starre und flexible Endoskope bekannt, die mit unterschiedlichsten Werkzeugen und optischen Einheiten ausgerüstet sind. In der Humanmedizin werden Endoskope für die Diagnostik als auch für minimal-invasive operative Eingriffe verwendet.

In jüngerer Zeit werden im Rahmen neuartiger Diagnose- und Therapiemethoden technische Trägersysteme benötigt, mit deren Hilfe im Peritonealraum an Organen Gewebeproben entnommen werden, Spezialsensoren für die *in-vivo* Gewebeanalyse positioniert werden, personalisierte Medikamente punktgenau an geschädigten Organen platziert oder injiziert werden, Drainagen gelegt oder sonstige Gewebe-Manipulationen durchgeführt werden können.

Die bislang verfügbaren Lösungsansätze basieren entweder auf konventionellen Chirurgiemethoden oder nutzen technische Assistenzsysteme der minimalinvasiven Chirurgie (MIC). Hierbei handelt es sich entweder um manuell geführte Instrumente der Laparoskopie (Werkzeuge und Kamera) oder um Instrumente, die durch ein robotisches Trägersystem geführt werden (z. B. DaVinci-Robotersystem). Bei beiden MIC-Verfahren muss der Bauchraum des Patienten großvolumig mit CO₂ befüllt und aufgeblasen werden, um für die Instrumentenmanipulation Platz zu schaffen. Dies belastet den Patienten erheblich.

Eine weitere Zugangsmöglichkeit besteht durch den Einsatz spezieller Kathetersysteme, deren Spitzen durch den Chirurgen manuell (direkt oder antriebstechnisch unterstützt) vorgeschoben und gelenkt werden müssen. Bei dieser Technik erfolgt der Zugang über Körperöffnungen und/oder Gefäße und die Instrumentennavigation wird durch Bildgebungsverfahren kontrolliert. Die Navigation im Peritonealraum zwischen den Organen und zwischen den Bindegewebsschichten ist mit dieser Methode schwierig und in bestimmten Bereichen nicht möglich. Bei den bekannten technischen Unterstützungssystemen sind die Antriebssysteme für die Bewegungserzeugung extern angeordnet und der Aufwand für die sichere Bewegungsübertragung ist hoch.

Die WO 2018/154326 A1 zeigt eine robotische Vorrichtung, welche aus mehreren, von elastischem Material bedeckten Segmenten besteht. Die Segmente ermöglichen eine Verkürzung und Verlängerung, eine Verbiegung sowie eine Vorwärts- und Rückwärtsbewegung.

Aus der KR 101009053 B1 ist ein Wartungs- und Bewegungssystem für einen Mikroroboter für die intravaskuläre Therapie bekannt. Das System umfasst Bewegungs- und Halteelemente sowie Elemente zum Verfahren des Mikroroboters.

Die US 6 702 734 B2 beschreibt einen selbstfahrenden endoskopischen Mikroroboter und ein System für die Darm-Endoskopie. Der Mikroroboter umfasst bogenförmige Stützarme, welche als Gehäuse dem Schutz dienen. Die Stützarme sind aus einem flexiblen Material.

Die WO 2021/163615 A1 beschreibt ein mikrorobotisches System in Schlauchform, welches durch ein Medium in seiner Flexibilität steuerbar ist, wobei das Medium in longitudinalen Kanälen geführt ist. Das beschriebene Robotersystem umfasst ein robotergestütztes chirurgisches Werkzeug und ein Lenksystem, das so konfiguriert ist, dass es das robotergestützte chirurgische Werkzeug auf der Grundlage von Bewegungswinkelbefehlen entlang der X- und Y-Achsen steuert. Eine Rechenvorrichtung, die ein Programm mit künstlicher Intelligenz ausführt, ist konfiguriert, um Bewegungswinkelbefehle auf der Grundlage einer aktuellen Position des robotergestützten chirurgischen Werkzeugs zu berechnen.

Die US 2009/0093675 A1 beschreibt ein medizinisches System für den Zugang über eine Körperöffnung. Das System umfasst ein medizinisches Instrument und einen Übertubus. Der Übertubus definiert ein Übertubuslumen, das so bemessen ist, dass es das medizinische Instrument aufnehmen kann. Der Übertubus hat ein distales Ende mit einem verjüngten Abschnitt und einer Vielzahl von sich in Längsrichtung erstreckenden Schlitzen, die eine Vielzahl von Klappen bilden. Der sich verjüngende Abschnitt des Übertubus ist relativ zum medizinischen Instrument so bemessen, dass eine Verschiebung des medizinischen Instruments durch das distale Ende des Übertubus die Vielzahl von Klappen zwingt, sich radial nach außen zu bewegen.

Die WO 2021/207574 A1 zeigt eine Endoskop-Kupplungsvorrichtung mit einem proximalen Ende, das zur Befestigung an einem Arbeitsende eines Endoskops konfiguriert ist, und einem Visualisierungsabschnitt, der die Betrachtung von Gewebe ermöglicht. Die Kupplungsvorrichtung umfasst weiterhin einen Instrumentenkanal mit einem offenen distalen Ende und einem proximalen Ende, das zur Ausrichtung mit einem Arbeitskanal des Endoskops konfiguriert ist. Die Kupplungsvorrichtung ist so konfiguriert, dass sie ein Instrument, das den Instrumentenkanal durchquert hat, festhält und/oder sichert, um sicherzustellen, dass das Instrument an der Zielstelle verbleibt und/oder um den Austausch des Instruments zu erleichtern.

Die DE 10 2020 107 309 A1 betrifft ein Trägersystem umfassend einen Zentraltubus mit einem proximalen und einem distalen Ende, einer innerhalb des Zentraltubus angeordneten Aufnahme und mindestens einem an der Außenseite des Zentraltubus angeordneten expandierbaren Fixierelement, welches einen kontrahierten und einen expandierten Zustand einnehmen kann. Der Zentraltubus ist zumindest an einem Ende fluiddicht durch ein Abschlusselement verschlossen. Zwischen dem Fixierelement und dem Zentraltubus verbleibt ein Durchlass auch im expandierten Zustand des Fixierelements.

Die US 2019/0159916 A1 beschreibt eine Vorrichtung zum Einführen in ein Gefäß, die zur Durchführung einer Translationsbewegung durch das Gefäß geeignet ist. Die Vorrichtung umfasst einen flexiblen Schlauch, der ein Translationsstellglied enthält. Verankerungsaktuatoren sind entlang der Länge des flexiblen Schlauchs angeordnet und so konfiguriert, dass sie sich in Abstimmung mit der Ausdehnung und Kontraktion des Translationsaktuators steuerbar gegen die Innenwand des Gefäßes ausdehnen, um eine Translationsbewegung zu bewirken.

Aus der US 2007/179339 A1 ist eine endoskopische Vorrichtung bekannt, zur autonomen Fortbewegung durch eine Körperhöhle. Die Vorrichtung umfasst einen röhrenförmigen Körper, der aus einem elastischen Material hergestellt ist und sich zwischen zwei Endabschnitten erstreckt. Der röhrenförmige Körper enthält einen Anker zur vorübergehenden und abwechselnden Befestigung der Endabschnitte an einem Wandabschnitt des Körperhohlraums, synchron mit den entsprechenden axialen Ausdehnungen und Kontraktionen des röhrenförmigen Körpers. Der rohrförmige Körper weist eine über seine Länge verteilte Verstärkungsstruktur auf, die in radialer Richtung im Wesentlichen starr ist und in axialer Richtung nachgibt, wobei die Verstärkungsstruktur entweder eine Vielzahl relativ starrer Ringe oder mindestens eine Schraubenfeder umfasst.

Die US 2020/305796 A1 beschreibt einen Mikroroboter, der so konfiguriert ist, dass er sich in einem viskosen Material bewegen kann, insbesondere in einem Organ eines Subjekts. Der Mikroroboter weist eine Antriebsstruktur auf, die einen Kopfabschnitt, einen hinteren Abschnitt und einen verformbaren Abschnitt, der den Kopfabschnitt und den hinteren Abschnitt verbindet, umfasst. Der verformbare Teil ist entlang einer Hauptachse, die den Kopfteil und den hinteren Teil verbindet, durch Dehnung/Kontraktion verformbar. Die Antriebsstruktur umfasst ferner einen Motor, der so konfiguriert ist, dass er sequenzielle Dehnungs-/Kontraktionszyklen des verformbaren Teils auslöst.

Aus der JP H02 141657 A ist eine in einem Rohr selbstfahrende Vorrichtung bekannt. Für einen stabilen Eigenantrieb ist sowohl an der vorderen als auch an der hinteren Endseite eines elastischen Antriebskörpers ein Ballon vorgesehen, der durch ein Fluidrohr gespeist und entladen wird und sich in axialer Richtung ausdehnt und zusammenzieht. Weiterhin sind harte rutschfeste Vorsprünge an den Außenflächen der Ballons angeordnet.

Die US 2020/221938 A1 zeigt einen selbstfahrenden Roboterkörper mit einem Rohr und einer Antriebsstruktur, die eine erste Antriebseinheit, eine zweite Antriebseinheit und eine dritte Antriebseinheit umfasst. Die Antriebseinheiten sind gleichmäßig an einer Umfangswand des Rohrhohlraums entlang der Achse des Rohrs befestigt. Weiterhin sind mindestens zwei Stützstrukturen vorgesehen, wobei jede der zwei benachbarten Stützstrukturen mindestens eine dazwischen angeordnete Antriebsstruktur aufweist. Die Stützstrukturen sind fest mit der Antriebsstruktur verbunden und an der Umfangswand des Rohrhohlraums angeordnet.

Die US 5,662,587 betrifft einen Roboter zur Durchführung endoskopischer Eingriffe in flexiblen und gekrümmten menschlichen oder tierischen Lumen. Dafür sind eine Vielzahl von Segmenten miteinander verbunden. Zugsegmente umschließen die Lumenwände, andere Segmente enthalten Aktuatoren, die das Endoskop veranlassen, seine Form durch Biegen, Strecken oder eine Kombination aus Biegen und Strecken lokal zu verändern. Die Segmente können so gesteuert werden, dass eine wurm- oder schlangenartige Fortbewegung durch ein gekrümmtes und flexibles Lumen ermöglicht ist. Eine am hinteren Segment angebrachte Druckgasleitung liefert komprimiertes Gas zur Insufflation des Lumens und kann optional zum Antrieb der Aktuatoren verwendet werden, die den Betrieb der Endoskopsegmente steuern. Das Führungssegment kann Fernsehkameras, Ultraschallwandler, Biopsiearme, Arzneimittelverabreichungssysteme oder andere Sensoren, diagnostische Hilfsmittel, therapeutische Geräte und chirurgische Werkzeuge enthalten. Medizinische Instrumente und Sensoren können auch in den hinteren oder mittleren Segmenten untergebracht werden.

Aus der DE 102 09 986 B4 ist ein Endoskopschaft bekannt mit einem beweglichen distalen Endabschnitt, der mittels einer Betätigungsvorrichtung abkrümmbar ist. Der distale Endabschnitt besitzt ein dezentral angeordnetes, sich längs erstreckendes Schlauchelement, das aus einer Mehrzahl von unmittelbar übereinander angeordneten Faltenbälgen einstückig gebildet ist und dabei einen durchgehenden Druckraum ausbildet, um sich bei Druckbeaufschlagung in Längsrichtung auszudehnen.

Die US 2011/0054253 A1 beschreibt eine Vorrichtung zur automatisierten Translationsbewegung eines Endoskops durch den Verdauungstrakt. Die Vorrichtung umfasst pneumatische Translationsbewegungsmittel und elektronische Steuermittel, die das automatisierte und koordinierte Verfahren der Translationsbewegungsmittel ermöglichen. Die Mittel zur Translationsbewegung umfassen: eine radiale Kammer, die auf dem Endoskop fixiert ist; eine balgförmige axiale Kammer, die über ein Ende mit dem Körper des Endoskops verbunden ist; eine radiale Kammer, die auf dem Endoskop schwimmt und am zweiten Ende der balgförmigen axialen Kammer befestigt ist.

Die US 4,176,662 zeigt ein Endoskop mit einem Antriebsmechanismus und mindestens einem Sender am distalen Ende, der Energiewellenstöße zur Verfolgung der Position des distalen Endes durch die Verwendung von zwei oder mehr Wandlern auf den anterioren oder lateralen Oberflächen eines Patienten überträgt. Der Antriebsmechanismus kann aus zwei radial ausdehnbaren Blasen bestehen, die durch einen axial ausdehnbaren Balg getrennt sind, wobei nur die vordere Blase am distalen Ende befestigt ist, so dass durch Ausdehnen und Zusammenziehen der Blasen in der richtigen Reihenfolge der Antrieb des Endoskops erreicht wird. Es zeigt sich aber, dass der beschriebene ausdehnbare Balg nicht nur axial seine Abmessungen ändert, wenn dafür ein Medium zugeführt bzw. Abgeführt wird, sondern auch seine radiale Abmessung, was zu einer Behinderung der Fortbewegung führt. Auch erweist sich als nachteilig, dass die radial ausdehnbaren Blasen beim Zusammenziehen nur eine geringe Reduktion ihres Umfangs zeigen, sodass sie für unerwünscht hohe Reibungskräfte beim gewünschten axialen Vortrieb des Endoskops verantwortlich sind.

Ausgehend vom Stand der Technik besteht eine Aufgabe der vorliegenden Erfindung darin, eine verbesserte mikrorobotische Einheit bereitzustellen, die in technischen und insbesondere in organischen Hohlräumen bzw. Organfalten fortbewegt und positioniert werden kann. Diese Einheit soll eine einfache und sichere Navigation in Hohlräumen, insbesondere im Peritonealraum zwischen den dort befindlichen Organen gestatten. Die Einheit soll außerdem mit unterschiedlichen Werkzeugen, Sensoren und/oder Beobachtungselementen ausrüstbar sein, um diese an einen vorbestimmten Ort zu verbringen. Insbesondere soll die Bewegung der Einheit in einer nachgiebigen Umgebung mit rutschigen, empfindlichen Oberflächenschichten ermöglicht werden, ohne diese umgebenden Schichten zu beschädigen, sodass in organischen Hohlräumen die Verletzung von Gewebe weitgehend vermieden wird. Die Einheit soll außerdem so gestaltet sein, dass selbst im Fehlerfall, beispielsweis beim ungewollten Austreten eines Versorgungsmediums, keine Gefährdung oder Beschädigung der umliegenden Gewebebereiche droht.

Diese Aufgabe wird gelöst durch eine mikrorobotische Einheit gemäß dem beigefügten Anspruch 1. Besondere Ausführungsformen der erfindungsgemäßen Einheit sind in der nachfolgenden Beschreibung und in den Unteransprüchen genannt.

Die erfindungsgemäße mikrorobotische Einheit zeichnet sich zunächst dadurch aus, dass das vordere und das hintere Spreizmodul jeweils eine äußere Ballonhülle besitzen, deren Ausdehnung sich bei Zufuhr des Versorgungsmediums erweitert und sich bei Abfuhr des Versorgungsmediums zusammenzieht. Der resultierende Umfang der Ballonhülle wird dabei durch in ihr erzeugten Druck bzw. Unterdruck gesteuert. Bevorzugt besitzen die Module der mikrorobotischen Einheit einen kreisrunden Querschnitt und eine axiale Erstreckung, sodass die Einheit insgesamt eine zylindrische Form aufweist, wobei der Durchmesser des Zylinders vorzugsweise im Bereich von 0,5 bis 3 cm und die Gesamtlänge im Bereich von 1 bis 10 cm liegen. An der Außenseite jeder Ballonhülle der Spreizmodule ist eine Oberflächenstrukturierung vorgesehen, die im ausgedehnten Zustand eine temporäre Verankerung/Fixierung im umgebenden Gewebe bewirkt, ohne dass es zu einer Beschädigung des Gewebes kommt. Die Oberflächenstrukturierung der Ballonhülle besitzt mehrere in radialer Richtung der Ballonhülle ausdehnbare Kuppeln, auf denen Borsten ausgebildet sind, die ebenfalls in radialer Richtung der Ballonhülle ausdehnbar sind.

Ebenso können strukturbildende Elemente auf die zunächst glatte Oberfläche der Ballonhülle aufgesetzt werden.

Das zwischen den Spreizmodulen befindliche Schubmodul der mikrorobotischen Einheit besitzt eine Faltenbalgstruktur, die bei Zufuhr des Versorgungsmediums eine axiale Ausdehnung und bei Abfuhr des Versorgungsmediums eine axiale Verkürzung des Schubmoduls bewirkt. Die Faltenbalgstruktur besitzt eine äußere, sich axial erstreckende Stützstruktur, welche eine Änderung der Ausdehnung der Faltenbalgstruktur in axialer Richtung gestattet, gleichzeitig eine Änderung der Ausdehnung in radialer Richtung begrenzt oder gänzlich verhindert. Die sich axial erstreckende Stützstruktur ist in der Art mehrerer aneinandergereihter Kniehebelstrukturen geformt, die in die Wandung der Faltenbalgstruktur integriert sein können oder aufgesetzt werden. Durch diese spezielle Balgstruktur wird ein energieoptimierter Vorschub erreicht.

Die Stützstruktur kann beispielsweise durch geeignet variierende Materialverteilung in der Faltenbalgstruktur erzeugt werden, um den Kniehebeleffekt zu erzielen. Alternativ oder ergänzend kann die Stützstruktur durch eine Aussteifung der Außenringe des Faltenbalges (z.B. partielle, steife Beschichtung) verstärkt und optimiert werden. Bei Druckbeaufschlagung des Faltenbalgs mithilfe des zugeführten Versorgungsmediums erfolgt dann praktisch keine Ausdehnung im Faltenbalgdurchmesser. Da für das radiale Zusammenziehen des Faltenbalgs vorzugsweise Unterdruck in diesem erzeugt wird, kann durch die konkrete Stützstruktur ein "Zusammenfallen" des Faltenbalgs dennoch verhindert werden.

Vorzugsweise umfasst die mikrorobotische Einheit mehrere Steuerelemente, welche die Zu- und Abfuhr des Versorgungsmediums zu den einzelnen Spreizmodulen und zum Schubmodul in Abhängigkeit von Steuersignalen steuern. Die Steuerelemente können in der Einheit selbst oder entfernt von dieser im Bereich der Zufuhr des Versorgungsmediums positioniert sein.

Wesentliche Vorteile dieser erfindungsgemäßen Einheit bestehen darin, dass eine sehr flexible Navigation der Einheit im Peritonealraum zwischen den Organen möglich wird. Die Zu- und Abfuhr des Versorgungsmediums gestattet das Festlegen der Einheit an einem beliebigen Ort, die Erzeugung eines Vorschubs, ebenso wie eine Richtungsänderung und eine Rückwärtsbewegung, jeweils in einer unstrukturierten, nachgiebigen Umgebung. Dies gestattet ein flexibles Manövrieren in einer nachgiebigen Umgebung mit rutschigen Oberflächenschichten. Somit ist auch das Umgehen von Gewebehindernissen im Peritonealraum möglich und eine Beschädigung von Organen, Gefäßen, Nervensträngen etc. lässt sich vermeiden. Die Kontrolle der Position und der Lage der Einheit kann mit geringem gerätetechnischem Aufwand erfolgen. Da die mikrorobotische Einheit selbst zur Bewegung aktuiert wird, entfällt der Antrieb über einen aufwendigen nachgiebigen Stabmechanismus, wodurch auch das umgebende Gewebe geschont wird.

Durch das am Faltenbalg angewandte Kniehebelprinzip wird ein besonders gutes Verhältnis von Druck in der Faltenbalgkammer zu realisiertem Vorschub erzielt, sodass der benötigte Vorschub bereits mit geringen Druckänderungen erreicht wird. Dies dient auch der verbesserten Betriebssicherheit bei Nutzung der mikrorobotischen Einheit, da selbst im Fehlerfall, z.B. wenn die Einheit beschädigt werden sollte, keine Verletzungsgefahr für die umgebenden Organe oder dergleichen durch unter hohem Druck stehende Versorgungsmedien besteht.

Der Vorschub- und die Lenkung der mikrorobotischen Einheit erfolgen mittelbar durch eine alternierend wirkende, lenkbare Schubbewegung, die in beiden Richtungen Schubkräfte aufbauen kann und durch das Schubmodul erzeugt wird. Das Versorgungsmedium kann je nach Ausführungsform flüssig oder gasförmig sein, sodass ein hydraulisches oder pneumatisches Kraftübertragungssystem resultiert. Es versteht sich, dass Versorgungsleitungen, Steuerelemente usw. an das gewählte Versorgungsmedium angepasst sind. Der eigentliche Schub in axialer Richtung der Einheit wird durch das Schubmodul erzeugt, welches zwischen den Spreizmodulen angeordnet ist. Die Spreizmodule dienen zur temporären Fixierung, indem ihre jeweilige Ballonhülle hydraulisch oder pneumatisch ausgedehnt wird, d. h. ihren Umfang deutlich vergrößert, beispielsweise um den Faktor 1,5 bis 3. Die vom Schubmodul erzeugte Vorschubkraft wirkt gegen eine solche temporäre Fixierung eines der Spreizmodule, sodass je nach Wahl des fixierten vorderen oder hinteren Spreizmoduls eine Bewegung axial nach vorn oder hinten resultiert.

Gemäß einer bevorzugten Ausführungsform umfasst die Einheit einen Zentralkanal, der sich vom Versorgungsanschluss durch das hintere Spreizmodul und das Schubmodul hindurch bis zum vorderen Spreizmodul erstreckt. Außerdem sind bevorzugt mehrere Zusatzkanäle vorgesehen, über welche alle Module individuell oder gemeinsam mit dem Versorgungsmedium versorgt werden, vorzugsweise gesteuert über die Steuerelemente.

Besonders bevorzugt ist das vordere Ende des Zentralkanals durch einen Klappenmechanismus verschlossen, sodass Medien oder Werkzeuge aus dem Zentralkanal nach außen geführt oder externe Materialien in den Zentralkanal hineingezogen werden können. Durch den Zentralkanal können beispielsweise Sonden, Werkzeuge und Sensoren bis zur Spitze der mikrorobotischen Einheit geführt werden. Im Zentralkanal kann vorzugsweise ein elektrisch, hydraulisch oder pneumatisch schaltbarer Arretierungsmechanismus vorhanden sein, um Katheterwerkzeuge und Sonden temporär zu fixieren. Weitere Kanäle können vorgesehen sein, beispielsweise einen Kanal zur Zuführung des Hydraulik- oder Pneumatik-Mediums (Versorgungsmedium) sowie einen Kanal zur Zuführung von Spülflüssigkeit und/oder zur Abführung entsprechender Flüssigkeiten.

Bei einer vorteilhaften Ausführungsform ist in Schubrichtung der Einheit axial vor dem vorderen Spreizmodul ein kegelstumpfförmiger Dorn angebracht. Dieser besitzt besonders bevorzugt eine zentrale Durchtrittsöffnung, durch welche weitere Werkzeuge/Instrumente aus der Einheit heraus geschoben werden können, beispielsweise Klingen, Klemmen oder Sensoren. Beim Vorschub der Einheit in den Organfalten bzw. zwischen einzelnen Organen durch das diese umgebende Bindegewebe müssen vorhandene Bindegewebsfasern von der Einheit durchdrungen werden. Dies erfolgt durch eine nicht beanspruchte Methode der stumpfen Präparation, d. h. ein Aufreißen bzw. Auseinanderdrängen der Gewebefasern, vergleichbar der Methode bei konventionellen OP-Techniken. Dies wird durch den stumpfen Dorn am vorderen Ende der Einheit ermöglicht.

Die mikrorobotische Einheit wird beispielsweise durch eine kleine chirurgisch geschaffene Öffnung in der Bauchdecke eingeführt. Auf der Basis vorhandener 3D-Daten vom Peritonealraum, die durch CT, MRT oder Ultraschall gewonnen werden, kann der gewünschte Pfad zum Zielpunkt berechnet werden, vorzugsweise unter Nutzung von Systemen, die auf Künstlicher Intelligenz (KI) basieren. Die Bewegung der mikrorobotischen Einheit kann vorzugsweise mit Hilfe eines referenzierten externen 3D-Ultraschall-Messsystems kontrolliert und verfolgt werden.

Gemäß einer vorteilhaften Ausführungsform umfasst die mikrorobotische Einheit einen oder mehrere Sensoren, die beispielsweise in einem der Spreizmodule integriert oder in angekoppelten Modulen angeordnet sind. Gemäß einer weitergebildeten Ausführungsform besitzt die mikrorobotische Einheit ein angekoppeltes Schlauchsystem, welches an den Versorgungsanschluss angeschlossen ist. Über dieses Schlauchsystem kann die Einheit mit elektrischer Energie und/oder mit dem pneumatischen oder hydraulischen Versorgungsmedium versorgt werden. Weiterhin können über das Schlauchsystem Diagnose- oder Therapiesonden zugeführt werden oder es können flüssige/gasförmige Medien zu- oder abgeführt werden.

Gemäß weiterer bevorzugter Ausführungsformen bildet die mikrorobotische Einheit eine mobile Trägerplattform für Sensoren (z. **B.** zur *in-situ* Gewebeanalyse) oder Mikrowerkzeuge zur Probennahme sowie spezielle Werkzeuge zur Gewebebeeinflussung vor Ort (Ablationswerkzeuge, Absetzten von Medikamenten oder Platzieren von elektronischen Kapseln, **z. B.** zur Beeinflussung von körpereigenen Signalleitungen). Die Einheit kann je nach Anwendungsfall mit jeweils unterschiedlichen Sensoren oder Werkzeugen oder dergleichen ausgerüstet werden.

Eine abgewandelte Ausführungsform zeichnet sich dadurch aus, dass mehrere mikrorobotische Einheiten über ein gemeinsames Schlauchsystem versorgt werden und auf diese Weise miteinander gekoppelt sind. Ebenso ist es möglich, dass eine Einheit mehr als zwei Spreizmodule und/oder mehr als ein Schubmodul umfasst. Eine nochmals abgewandelten Ausführungsform verwendet mehrere, beispielsweise drei oder vier parallel geschaltete Einheiten, die jeweils mit einem separaten Schlauchsystem versorgt werden.

Da die Spreiz- und Schubmodule über die Steuerelemente in wechselnder Folge über das Versorgungsmedium aktivierbar sind, lassen sich viele verschiedene Betriebszustände einstellen. So ist es möglich, die gesamte Einheit in einer Vorschubrichtung vorwärts aber auch wieder rückwärts zu bewegen, um die Einheit nach Abschluss einer Behandlungsprozedur wieder aus dem Hohlraum herauszuführen. Die Rückwärtsbewegung kann durch Zug am Schlauchsystem unterstützt werden. Ebenso kann die Einheit durch Aufweiten beider Spreizmodule an einem Behandlungsort zeitweise fixiert werden.

Eine vorteilhafte Ausführungsform der Einheit besitzt weitere Module, die axial hinter dem hintern Spreizmodul angekoppelt sind. Diese weiteren Module können beispielsweise Speichermodule für Medikamente oder für Versorgungsenergie sein.

Vorteilhaft ist eine Ausführungsform, die eine Kamera umfasst, welche für die Beobachtung der Umgebung der mikrorobotischen Einheit geeignet ist. Beispielsweise kann die Kamera am kegelstumpfförmigen Dorn angeordnet sein, um optische Aufnahmen des vor der Einheit liegenden Bereichs zu liefern.

Gemäß einer weitergebildeten Ausführungsform kann die Einheit andere Sensoren zur Erfassung der Umgebungs- und Kontaktparameter sowie Sensoren zur Erfassung der Lage- und Bewegungszustände umfassen.

Bei einer abgewandelten Ausführungsform wird das Versorgungsmedium nicht über das Schlauchsystem zugeführt, sondern stattdessen elektrische Energie, die der Einheit zugeführt wird. In diesem Fall umfasst die Einheit eine oder mehrere Pumpen, welche innerhalb der Einheit ein Versorgungsmedium zu den einzelnen Modulen transportieren, beispielsweise um die Spreizmodule aufzuweiten. Ebenso ist eine Kombination von internem und extern zugeführtem Versorgungsmedium möglich.

Gemäß einer Ausführungsform umfasst die Faltenbalgstruktur als Stützstruktur eine Gitterstruktur aus Kunststoff oder Metall, die in eine bevorzugt elastische Kunststoffhülle (z. **B.** aus Silikon) eingebettet ist. Die Gitterstruktur kann bevorzugt als sogenannte Nitinol-Struktur (Nickel-Titan-Legierung) oder eine vergleichbare Formgedächtnis-Legierung gebildet sein.

Die erläuterte Gestaltung des Schubmoduls und seiner Bestandteile ermöglicht es, dass der aufgebaute Druck in der Faltenbalgstruktur optimal in eine Vorschubbewegung umgesetzt wird, ohne Energieverluste für die Aktuierung der Eigenstruktur (z. B. Überwindung von intrinsischen Elastizitäten). Insbesondere bewirkt ein steigender Innendruck im Schubmodul eine Kraftwirkung sowohl an den Wirkflächen in Schubrichtung, als auch an den umschließenden Wandflächen durch das erwähnte Kniehebelprinzip, sodass eine Bewegungs- und Kraftwirkung in Schubrichtung erzeugt wird. Alternativ zum Kniehebelprinzip kann in abgewandelten Ausführungen auch das Prinzip der sogenannten Nürnberger Schere (Gelenkkette aus mehreren gekreuzten Stäben) eingesetzt werden. Vorzugsweise bleibt der Außendurchmesser der Faltenbalgstruktur bei Druckbeaufschlagung (von innen) konstant bzw. wird nicht über ein vorbestimmtes Maß überschritten.

Gemäß einer bevorzugten Ausführungsform kann das zentrale Balgelement der Faltenbalgstruktur durch eine äußere Schraubenfeder oder andere in Längsrichtung elastische Strukturen umgeben bzw. abgeschirmt sein. Die äußere Schraubenfeder erstreckt sich koaxial zur Faltenbalgstruktur.

Diese Schraubenfeder kann drei vorteilhafte Funktionen erfüllen: die Sicherstellung der Basis-Geradführung in der Schubnormalen, die einfache Führung von seitlichen Lenkseilen, welche der Ausdehnungsbeschränkung und Lenkung dienen, und die Verhinderung einer übermäßigen Durchmesserausdehnung bei unbeabsichtigtem Überdruck.

Gemäß einer bevorzugten Ausführungsform ist im Inneren des Faltenbalgelements eine leicht dehnbare Schraubenfeder angeordnet, welche mit einer druckdichten und elastischen Folie umgeben ist. Durch diese innere Schraubenfeder können weitere Funktionselemente nach dem Katheterprinzip geführt werden (Sonden, Werkzeuge etc.).

Die Schubausdehnung am Schubmodul erfolgt durch die Druck- bzw. Volumenbeaufschlagung des in der Faltenbalgstruktur befindlichen Versorgungsmediums. Zum Zusammenziehen wird im umgekehrten Fall ein relativer Unterdruck erzeugt.

Bevorzugt wird ein Versorgungsmedium verwendet, das über eine Pumpe aktuiert wird und aus einem geschlossenen Reservoir gespeist wird. Bei Erzeugung eines Unterdrucks wird das Versorgungsmedium in das Reservoir zurückgeführt. Das Reservoir kann ein Kolben-Zylinder-System, ein elastischer Speicherkörper oder eine weitere mikrorobotische Einheit sein. Bei einer externen Druckbereitstellung wird aufgrund der zu erwartenden Länge des Schlauchsystems ein pneumatisches Versorgungsmedium (z. B. Stickstoff) bevorzugt.

Gemäß einer besonders bevorzugten Ausführungsform kann eine Lenkung der mikrorobotischen Einheit erfolgen, indem die Vorschubbewegung abweichend von der Zentralachse der Einheit erzeugt wird. Diese Lenkung wird vorzugsweise durch eine temporäre Fixierung der seitlichen Dehnbarkeit der Faltenbalgstruktur des Schubmoduls erreicht. Dazu sind an der Faltenbalgstruktur Lenkelemente, insbesondere Lenkseile oder Lenkfedern angebracht, die sich axial erstrecken und durch Klemmelemente zeitweise blockiert werden können. Um die Lenkung zu aktivieren, genügt es, wenn das Lenkseil oder die Lenkfeder festgeklemmt wird. Durch die derart bewirkte temporäre Verhinderung von seitlichen Balgausdehnungen wird deutlich weniger Energie benötigt, als bei einer aktiven Seillenkung.

Besonders bevorzugt sind zur zuverlässigen Lenkung 2 x 2 antagonistische Lenkseile vorgesehen. Die Lenkseile werden vorzugsweise durch ein elastisches Element straff gehalten. Als Lenkseil-Klemmmechanismen können Kraftschluss-, Formschluss-, Keil- und Schlingprinzipien genutzt werden, welche elektrisch und/oder durch ein Fluid geschaltet werden.

Bei einer abgewandelten Ausführungsform sind die Lenkelemente durch an der Faltenbalgstruktur aufgebrachte, aktivierbare Versteifungsschichten gebildet. Die Versteifungsschichten können beispielsweise durch einen elektrischen Strom oder durch eine Temperaturänderung aktiviert, d. h. in ihrer Steifigkeit beeinflusst werden, um somit einseitig die Ausdehnung des Faltenbalgs zu blockieren bzw. zu reduzieren.

Das mindestens eine vordere und eine hintere Spreizmodul haben jeweils die Aufgabe, im gespreizten Zustand die mikrorobotische Einheit in ihrer Lage zu fixieren und/oder ein Gegenlager bei der Erzeugung einer Vorschub- oder Rückzugskraft zu erzeugen. Hierbei wird ein Optimum aus einer minimalen internen Krafterzeugung bei einer maximalen Verankerungswirkung angestrebt. Es versteht sich, dass mehr als zwei Spreizmodule an der Einheit vorgesehen sein können, die sich gegenseitig ergänzen und ggf. die Flexibilität der Einheit weiter steigern.

Gemäß einer hier nicht beanspruchten Ausführungsform ist die Oberflächenstrukturierung der elastischen Ballonhülle des jeweiligen Spreizmoduls als eine borstenartige Struktur gebildet. Die Borsten besitzen eine hinreichende Steifigkeit, um im aufgestellten Zustand eine hohe Reibungswirkung mit dem umliegenden Gewebe zu erzeugen (partieller Formschluss), und sind andererseits hinreichend weich, um bei einer Vorschubbewegung das umgebende Gewebe nicht zu verletzen (keine Mikrohaken). Alternativ zu den Borsten kann an der Ballonhülle eine Oberfläche mit einer sogenannten Kirigami-Struktur (Falten mit Einschnitten) eingesetzt werden.

Erfindungsgemäß ist die Oberflächenstrukturierung der elastischen Ballonhülle des jeweiligen Spreizmoduls durch eine kaskadierte Struktur gebildet. Insbesondere können an der Ballonhülle angebrachte Blasen ihrerseits aufgesetzte Noppen aufweisen. Diese Kaskadierung ermöglicht eine große Ausdehnung der Oberflächenstrukturierung bei eingespeistem Versorgungsmedium (Druckaufbau) und stellt gleichzeitig eine mechanische Struktur bereit, die einen optimalen Kontakt zum umgebenden Gewebe zur Maximierung der Haltekraft bewirkt. Andererseits wird bei erzeugtem Unterdruck in den Spreizmodulen die Halte- und Reibungswirkung gegenüber der Umgebungsstruktur komplett aufgehoben. Die an der Oberfläche der Ballonhülle befindlichen Blasen fallen komplett nach innen und liegen dann unter dem Grunddurchmesser des Spreizmoduls. Auch die Noppen werden definiert nach innen gelegt. Besonders bevorzugt sind vier radialen Noppen um eine die Zentralnoppe angeordnet. Somit wird der Schubwiderstand am durchmesserreduziertem Spreizmodul auf ein Minimum reduziert. Ein weiterer Vorteil dieser Ausführungsform ist, dass die schaltbare Bewegungshemmung unabhängig von der Bewegungsrichtung des Schubmoduls wirkt. In abgewandelten Ausführungsformen können die nach außen gerichteten Spitzen der Noppen mit weiteren bewegungshemmenden Strukturen versehen werden (z.B. Mikroborsten, Mikrokörnung).

Das Ausdehnen bzw. Zusammenfallen der Ballonhülle des Spreizmoduls erfolgt durch Erzeugen von Überdruck bzw. Unterdruck (hydraulisch oder pneumatisch) im Spreizmodul. Bevorzugt werden Form und Material der Ballonhülle so gewählt, dass diese ohne eigenen Bewegungswiderstand (z. B. durch Überwindung von intrinsischen Elastizitäten) ausgedehnt (aufgespannt) und durch Unterdruck auf einen minimalen Durchmesser zusammengezogen werden kann, um somit beim Vorschub einen minimalen Bewegungswiderstand zu bieten. Der Zufluss und Abfluss des Versorgungsmediums wird über Steuerelemente, insbesondere schaltbare Mikroventile gesteuert.

Gemäß einer bevorzugten Ausführungsform hat das vordere Spreizmodul neben der Verankerung bzw. Fixierung im Gewebe auch die Aufgabe der sogenannten stumpfen Präparation des sich in Bewegungsrichtung befindenden Bindegewebes. Diese Funktion wird durch den am vorderen Spreizmodul angeordneten Dorn und/oder durch eine spezielle Gestaltung der Ballonhülle des vorderen Spreizmoduls unterstützt.

Weitere Vorteile, Einzelheiten und Abwandlungen der Erfindung werden anhand bevorzugter Ausführungsformen nachfolgend beschrieben, unter Bezugnahme auf die Zeichnung. Es zeigen:
- Fig. 1: eine perspektivische Ansicht einer ersten Ausführungsform einer erfindungsgemäßen mikrorobotischen Einheit;
- Fig. 2: eine perspektivische Explosionsdarstellung der mikrorobotischen Einheit gemäß Fig. 1;
- Fig. 3: Seitenansichten der mikrorobotischen Einheit gemäß Fig. 1 in zwei unterschiedlichen Betriebszuständen;
- Fig. 4: drei Abbildungen einer Faltenbalgstruktur eines Schubmoduls;
- Fig. 5: eine prinzipielle Darstellung eines Lenkmechanismus der mikrorobotischen Einheit;
- Fig. 6: eine prinzipielle Darstellung der mikrorobotischen Einheit mit einem durchgehendem Zentralkanal;
- Fig. 7: eine perspektivische Explosionsdarstellung einer zweiten Ausführungsform der mikrorobotischen Einheit;
- Fig. 8: die Ausführungsform gemäß Fig. 7 in zwei unterschiedlichen Vorschubzuständen.

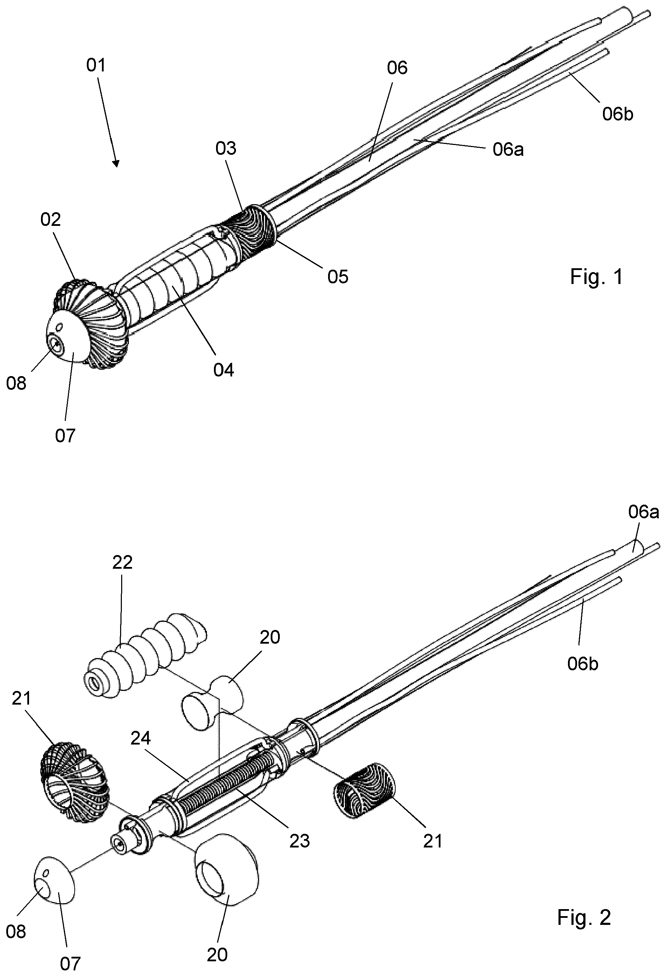

Fig. 1 zeigt in einer vereinfachten perspektivischen Ansicht eine erste Ausführungsform einer mikrorobotischen Einheit 01. Die Einheit 01 besitzt ein vorderes Spreizmodul 02 und ein hinteres Spreizmodul 03, die voneinander beabstandet und (im Ruhezustand) axial zueinander ausgerichtet sind. Zwischen den beiden Spreizmodulen 02, 03 erstreckt sich ein Schubmodul 04. Am hinteren Spreizmodul 03 befindet sich ein Versorgungsanschluss 05, an welchen ein sich axial erstreckendes Schlauchsystem 06 anschließt. Im dargestellten Beispiel umfasst das Schlauchsystem 06 einen Zentralkanal 06a sowie mehrere Zusatzkanäle 06b. In Vorschubrichtung vorn befindet sich am vorderen Ende der Einheit 01 ein kegelstumpfförmiger Dorn 07 mit einer vorderen Öffnung 08.

In Fig. 2 ist die mikrorobotische Einheit 01 in einer Explosionsdarstellung gezeigt. Daraus ist ersichtlich, dass sich mindestens der Zentralkanal 06a durch die Spreizmodule 02, 03 und das Schubmodul 04 erstreckt und am Dorn 07 endet. Auf diese Weise können z. B. miniaturisierte Werkzeuge bis zur vorderen Öffnung 08 im Dorn 07 geführt werden und aus dieser austreten. Die Zusatzkanäle 06b sind bis zu dem jeweiligen Modul geführt, um dieses mit einem Versorgungsmedium zu versorgen. Weiterhin sind Steuerelemente (nicht gezeigt) vorgesehen, welche die Zu- und Abfuhr des Versorgungsmediums zu den einzelnen Spreizmodulen und zum Schubmodul in Abhängigkeit von Steuersignalen steuern. Diese Steuerelemente können in die Einheit integriert sein oder aber an einem entfernten Ende des Schlauchsystems positioniert sein.

Die beiden Spreizmodule 02, 03 besitzen jeweils eine Ballonhülle 20, die mit Hilfe des Versorgungsmediums ausgedehnt oder zusammengezogen werden kann. In Fig. 1 und 2 ist das vordere Spreizmodul 02 und seine Ballonhülle im ausgedehnten Zustand gezeigt, während das hintere Spreizmodul 03 mit seiner Ballonhülle im zusammengezogenen Zustand gezeigt ist. Die Ballonhüllen 20 sind jeweils mit einer Oberflächenstrukturierung 21 versehen, die wie in Fig. 2 gezeigt als eigenständige Hülse gestaltet sein kann, oder in anderen Ausführungen integral mit der Ballonhülle ausgeführt ist, beispielsweise in Form von in die Ballonhülle integrierten Borsten, Noppen oder kaskadierten Strukturen. Im ausgedehnten Zustand bewirkt die Oberflächenstrukturierung 21 eine Verankerung im umgebenden Gewebe, ohne dass das Gewebe dadurch verletzt wird.

Weiterhin ist aus Fig. 2 ersichtlich, dass das Schubmodul 04 eine Faltenbalgstruktur 22 aufweist. Das zentrale Balgelement der Faltenbalgstruktur kann beispielsweise aus einem hochelastischen Silikon (z. B. im 3D-Druckverfahren) hergestellt werden. Die Dimensionierung erfolgt in Abhängigkeit von der Zielfunktionalität; beispielsweise können partielle Wandverdickungen zur Nachbildung einer Gitterstruktur vorgesehen sein. Im Inneren des Faltenbalgs 22 verläuft bei dieser Ausführungsform eine innere Schraubenfeder 23. An der Außenseite sind externe Versorgungskanäle 24 geführt.

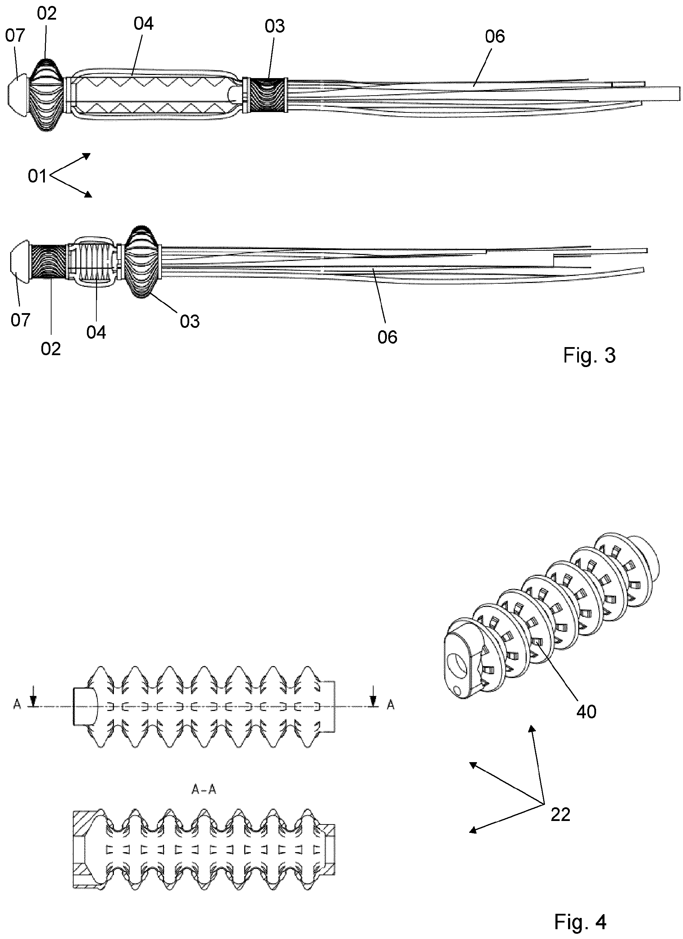

Fig. 3 zeigt die mikrorobotische Einheit 01 in zwei unterschiedlichen Betriebszuständen, um die Realisierung einer Vorschubbewegung zu verdeutlichen. In der oberen Abbildung ist das vordere Spreizmodul 02 durch Zufuhr des Versorgungsmediums ausgedehnt und besitzt somit einen gegenüber dem Schubmodul 4 deutlich größeren Außendurchmesser. Dem Schubmodul 4 ist ebenfalls Versorgungsmedium zugeführt, sodass es eine maximale Ausdehnung in axialer Richtung einnimmt, während sich sein Außendurchmesser aufgrund der Stützstruktur nicht wesentlich verändert. Dem hinteren Spreizmodul 03 ist das Versorgungsmedium entzogen, sodass seine Ballonhülle 20 zusammengezogen ist und einen verkleinerten Außendurchmesser (im Wesentlichen dem Durchmesser des Schubmoduls entsprechend) einnimmt.

In der unteren Abbildung der Fig. 3 befindet sich das vordere Spreizmodul 02 im zusammengezogenen Zustand, d. h. ihm wurde das Versorgungsmedium entzogen. Stattdessen befindet sich das hintere Spreizmodul 03 im ausgedehnten Zustand, d. h. ihm wurde Versorgungsmedium zugeführt und seine Ballonhülle 20 hat sich ausgedehnt, um im umliegenden Gewebe fixiert zu sein. Das Schubmodul 04 ist axial verkürzt, d. h. das Versorgungsmedium wurde entzogen. In zeitlicher Abfolge wurde zur Erreichung dieses Zustands zunächst die axiale Länge des Schubmoduls verkürzt, wodurch sich das hintere Spreizmodul (im zusammengezogenen Zustand) in Vorschubrichtung bewegt, wie es aus dem Vergleich der beiden Abbildungen in Fig. 3 gut ersichtlich ist. Im nächsten Schritt wird das hintere Spreizmodul 03 ausgedehnt, um es zu fixieren, und das vordere Spreizmodul 02 zusammengezogen, um es für die nächste Vorschubbewegung vorzubereiten. Damit ist der in der unteren Abbildung dargestellte Zustand erreicht. Im nachfolgenden Schritt (nicht gezeigt) wird dem Schubmodul 04 erneut Versorgungsmedium zugeführt, sodass es sich axial ausdehnt und das vordere Spreizmodul 02 in Vorschubrichtung nach vorn schiebt.

Fig. 4 zeigt eine Seitenansicht, eine Längsschnittansicht und eine perspektivische Ansicht der Faltenbalgstruktur 22 gemäß einer möglichen Ausführungsform. Für die Faltenbalgstruktur ist wesentlich, dass sie durch Zufuhr bzw. Abfuhr des Versorgungsmediums (Befüllen bzw. Entleeren des Faltenbalgelements) ihre axiale Ausdehnung ändert. Dazu können beispielsweise in der Außenwandung mehrere aneinandergereihte Kniehebel integriert sein. Bei der in Fig. 4 dargestellten Ausführungsform ist eine Gitterstruktur 40 in das Silikonmaterial des Faltenbalgelements eingebettet. Der Außendurchmesser des Balgsystems bleibt bei Druckbeaufschlagung im Wesentlichen konstant.

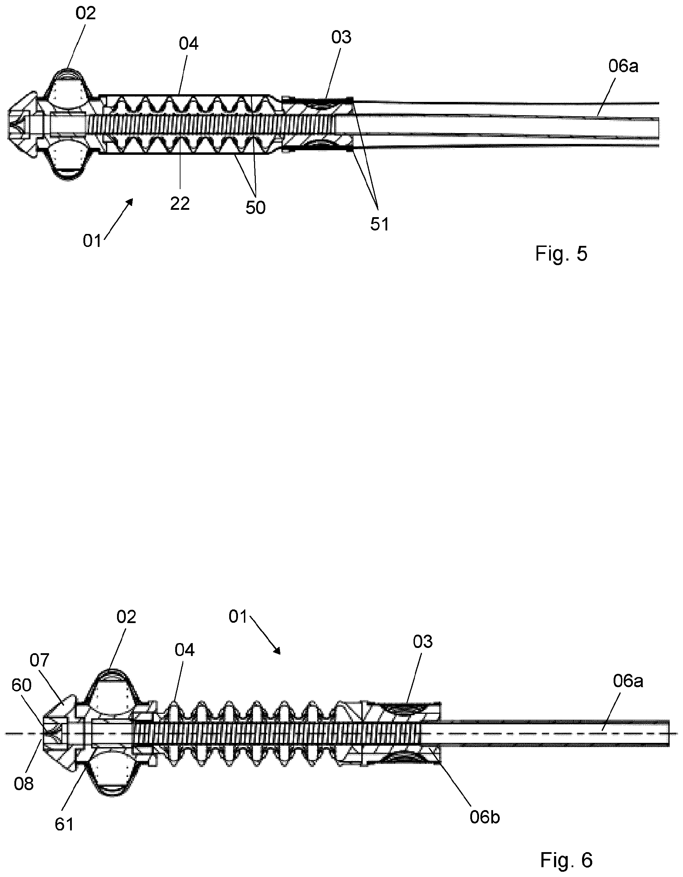

Fig. 5 zeigt in einer Schnittansicht den prinzipiellen Aufbau der mikrorobotischen Einheit 01 unter Berücksichtigung eines Lenkmechanismus. Generell lässt sich die Richtung der Vorschubbewegung ändern, indem auf einer axialen Seite des Schubmoduls 04 die axiale Ausdehnung kleiner gehalten wird als auf der gegenüberliegenden Seite. Im einfachsten Fall sind dazu mindestens zwei Lenkseile 50 als Lenkelemente an gegenüberliegenden Seiten des Balgelements 22 angeordnet. Jedem Lenkseil 50 ist ein Klemmelement 51 zugeordnet, bei dessen Aktivierung (z. B. mithilfe des Versorgungsmediums) das Lenkseil in einem verkürzten Zustand festgelegt werden kann. Dadurch kann sich das Balgelement 22 auf der Seite des verkürzten Lenkseils weniger axial ausdehnen, sodass die Vorschubbewegung in diese Richtung gekrümmt wird.

Fig. 6 zeigt in einer Schnittansicht nochmals Einzelheiten der mikrorobotischen Einheit 01 gemäß der zuvor beschriebenen Ausführungsform. Gut ersichtlich ist hier, dass der Zentralkanal 06a durch alle axial aneinandergereihten Module hindurch verläuft, bis zur vorderen Öffnung 08 im vorn liegenden Dorn 07. Der Zentralkanal 06a ist bei der gezeigten Ausführungsform am vorderen Ende durch innenliegende Klappen 60 verschlossen, die einerseits das Herausfahren von Werkzeugen oder die Abgabe von Infusionen oder dergleichen ermöglichen, andererseits das unerwünschte Eintreten von Flüssigkeit oder Gewebe verhindern. Im Zentralkanal 06a kann außerdem ein Arretierungsmechanismus 61 angeordnet sein, beispielsweise um ein chirurgisches Werkzeug festzulegen, wenn es über den Zentralkanal zugeführt wurde. Schließlich ist ersichtlich, dass über die Zusatzkanäle 06b gezielt Versorgungsmedium zu den einzelnen Modulen zuführbar bzw. abführbar ist, um dort unterschiedlichen Druck einzustellen, sodass in der oben beschriebenen Weise eine Vorschubbewegung initiierbar ist.

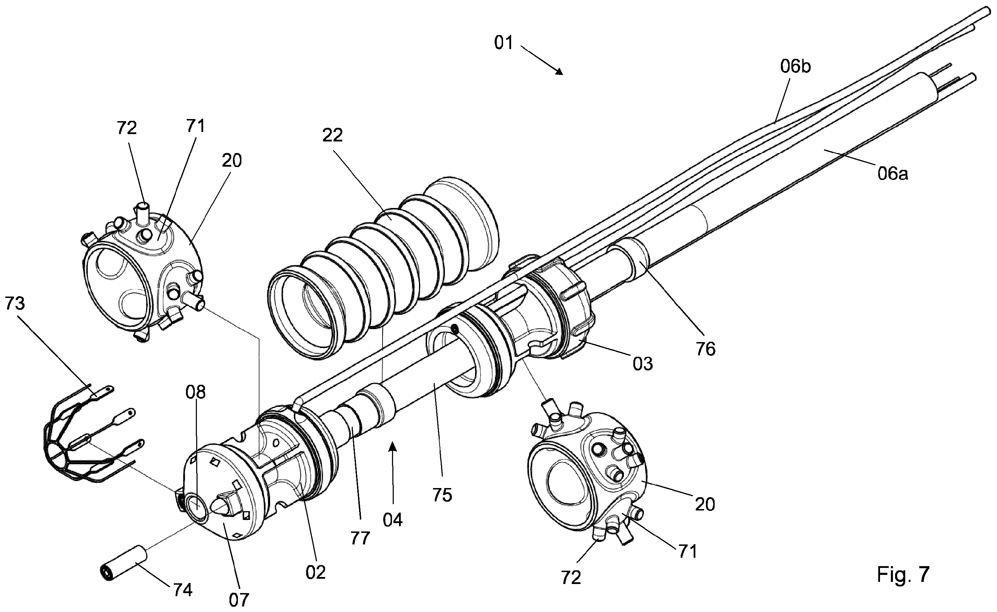

Fig. 7 zeigt in einer perspektivischen Explosionsdarstellung eine zweite Ausführungsform der mikrorobotischen Einheit 01. Das vordere Spreizmodul 02 und das hintere Spreizmodul 03 besitzen jeweils angepasste Ballonhüllen 20. Bei dieser Ausführungsform sind in die Ballonhüllen 20 mehrere kaskadierte Oberflächenstrukturierungen, die jeweils aus einer blasenartigen Kuppel 71 mit mehreren darauf angebrachten Borsten 72 bestehen. Die mehreren Kuppeln und ihre zugehörigen Borsten können sich bei Zufuhr des Versorgungsmediums radial nach außen erweitern, während andere Abschnitte des Grundkörpers der Ballonhülle 20 starr ausgebildet sein können und einen unveränderlichen Durchmesser besitzen. Die durch die Kuppeln und die Borsten gebildeten Oberflächenstrukturen wirken hierarchisch, d.h. bei Druckerhöhung im Spreizmodul werden je nach gewählter Materialsteifigkeit zunächst die Kuppeln 71 radial nach außen erweitert, woraufhin sich bei weiterer Druckerhöhung dann auf den Kuppeln 71 die Borsten 72 aufrichten bzw. ausfahren (oder in umgekehrter Reihenfolge, wenn die Materialien anders gewählt werden). Diese hierarchische Anordnung mehrerer Oberflächenstrukturen hat den Vorteil, dass bei nachfolgender Druckreduktion die Borsten 72 in die Kuppel 71 einklappen und dann mit der Kuppel weiter nach innen gezogen werden, somit nicht mehr über den minimalen Durchmesser der Ballonhülle 20 überstehen und beim weiteren Vorschub der Einheit 01 nur minimalen Reibungswiderstand an den Hohlraumwänden erzeugen.

In Fig. 7 ist außerdem ein Schutzgitter 73 dargestellt, welches über dem kegelstumpfförmigen Dorn 07 angebracht wird, um diesen und die dort angebrachten Einheiten zu schützen. Dazu gehört in dieser Ausführungsform eine Mikrokamera 74, welche im Dorn 07 positioniert ist.

In der in Fig. 7 gezeigten Ausführungsform verläuft zentral in der Einheit 01 ein starres oder biegsames Zentralrohr 75, welches einen Abschnitt des Zentralkanals 06a bildet. An seinem von der vorderen Öffnung 08 abgewandten Ende ist das Zentralrohr 75 durch eine leichtgängige und druckdichte Lagerung 76 geführt. Wenn durch die gesteuerte Druck-Unterdruck-Beaufschlagung der Faltenbalgstruktur 22 eine axiale Bewegung der Einheit veranlasst wird, gleitet der hintere Spreizkörper 03 auf dem Zentralrohr 75 und ist durch dieses geführt. Im Bereich der Faltenbalgstruktur 22 ist das Zentralrohr 75 vorzugsweise getrennt und mit einem elastischen Schlauchstück 77 verbunden. Somit ist das vordere Ende der Einheit 01 mit dem vorderen Spreizkörper 02 flexibel beweglich gegenüber dem nachfolgenden Abschnitt. Durch die Betätigung der innenliegenden Lenkseile kann das elastische Schlauchstück 77 partiell verkürzt werden, um bei der Vorschubbewegung eine Richtungsänderung des vorderen Spreizkörpers 03 mit dem Dorn 07 zu bewirken.

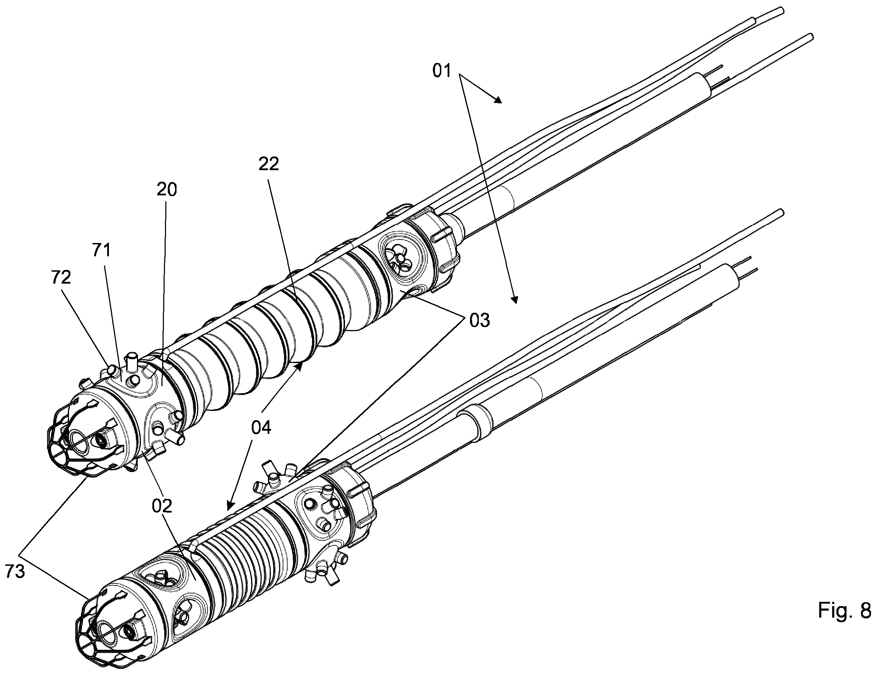

Fig. 8 zeigt die Ausführungsform gemäß Fig. 7 in zwei unterschiedlichen Vorschubzuständen in perspektivischer Ansicht. In der oberen Abbildung ist das in axialer Richtung vordere Spreizmodul 02 durch das Versorgungsmedium mit Druck beaufschlagt, sodass die Kuppel 71 und die Borsten 72 radial erweitert sind, um das vordere Spreizmodul im umschließenden Gewebe festzulegen. Das Schubmodul 04 ist mit Versorgungsmedium beaufschlagt, sodass die Faltenbalgstruktur 22 axial ausgedehnt ist, ohne in radialer Richtung ein wesentliche Querschnittsänderung zu erfahren. Das hintere Spreizmodul 03 ist nicht mit Überdruck beaufschlagt oder vorzugsweise an einen Unterdruck angeschlossen, sodass dessen Oberflächenstruktur in radialer Richtung minimiert ist, d.h. die Kuppel und die Borsten ragen nicht über den minimalen Außendurchmesser der Ballonhülle des hinteren Spreizmoduls hinaus. Um vom in der oberen Abbildung gezeigten Zustand in den unten abgebildeten Zustand zu gelangen, wird zunächst das Versorgungsmedium aus der Faltenbalgstruktur 22 abgepumpt, sodass sich dieses axial zusammenzieht, wodurch das hintere Spreizmodul 03 axial nach vorn verlagert wird.

In der unteren Abbildung der Fig. 8 ist das vordere Spreizmodul 02 nicht mit Druck beaufschlagt oder vorzugsweise an Unterdruck angeschlossen. Die Kuppel 71 und die Borsten 72 sind radial eingefahren. Somit nimmt das vordere Spreizmodul 02 seinen minimalen Querschnitt an. Das Schubmodul 04 ist in axialer Richtung zusammengezogen, entweder durch integrale Federkräfte und/oder durch Reduktion des Drucks des Versorgungsmediums. Das hintere Spreizmodul 03 ist mit Druck beaufschlagt, sodass Kuppel und Borsten radial nach außen gefahren sind, um das hintere Spreizmodul im umgebenden Gewebe festzulegen. Wenn im nächsten Schritt Versorgungsmedium zum Schubmodul 04 zugeführt wird, verlängert sich die Faltenbalgstruktur 22 des Schubmoduls 04, sodass das vordere Spreizmodul 02 axial nach vorn geschoben wird.

### Bezugszeichen

- 01: mikrorobotische Einheit
- 02: vorderes Spreizmodul
- 03: hinteres Spreizmodul
- 04: Schubmodul
- 05: Versorgungsanschluss
- 06: Schlauchsystem
- 06a: Zentralkanal
- 06b: Zusatzkanäle
- 07: kegelstumpfförmiger Dorn
- 08: vordere Öffnung im Dorn

- 20: Ballonhülle
- 21: Oberflächenstrukturierung an der Ballonhülle
- 22: Faltenbalgstruktur / zentrales Balgelement
- 23: innere Schraubenfeder mit elastischer Hülle
- 24: externe Versorgungskanäle

- 40: Gitterstruktur in der Faltenbalgstruktur
- 50: Lenkseile
- 51: Klemmelemente
- 60: Klappen im Zentralkanal
- 61: Arretierungsmechanismus

- 71: Kuppel
- 72: Borsten
- 73: Schutzgitter
- 74: Mikrokamera
- 75: Zentralrohr
- 76: Lagerung
- 77: elastisches Schlauchstück

## Patentansprüche

1. Mikrorobotische Einheit (01) zur Fortbewegung und Positionierung in organischen Hohlräumen, umfassend:
- ein vorderes Spreizmodul (02) und ein hinteres Spreizmodul (03), wobei jedes Spreizmodul (02, 03) mindestens eine äußere Ballonhülle (20) besitzt, deren Ausdehnung sich bei Zufuhr des Versorgungsmediums mindestens partiell erweitert, wobei an der Außenseite der Ballonhülle (20) eine Oberflächenstrukturierung (21) vorgesehen ist, die im ausgedehnten Zustand eine Verankerung im umgebenden Gewebe bewirkt;
- ein Schubmodul (04), welches zwischen den beiden Spreizmodulen (02, 03) angeordnet und mit diesen verbunden ist, wobei das Schubmodul (04) eine Faltenbalgstruktur (22) aufweist, die bei Zufuhr des Versorgungsmediums eine axiale Ausdehnung und bei Abfuhr des Versorgungsmediums eine axiale Verkürzung des Schubmoduls bewirkt, wobei die Faltenbalgstruktur (22) eine äußere, sich axial erstreckende Stützstruktur aufweist, um eine Änderung ihrer Ausdehnung in radialer Richtung zu verhindern oder zu begrenzen;
- einen Versorgungsanschluss (05), welcher sich axial am hinteren Spreizmodul (03) anschließt und der Zufuhr von mindestens einem Versorgungsmedium dient;
**dadurch gekennzeichnet, dass**
- die Oberflächenstrukturierung (21) der Ballonhülle (20) mehrere in radialer Richtung der Ballonhülle ausdehnbare Kuppeln (71) besitzt, auf denen Borsten (72) ausgebildet sind, die ebenfalls in radialer Richtung der Ballonhülle ausdehnbar sind;
- die sich axial erstreckende Stützstruktur der Faltenbalgstruktur (22) in der Art mehrerer aneinandergereihter Kniehebelstrukturen geformt ist.

2. Mikrorobotische Einheit (01) nach Anspruch 1, **dadurch gekennzeichnet, dass** sich ein Zentralkanal (06b) durch das hintere Spreizmodul (03) und das Schubmodul (04) bis zum vorderen Spreizmodul (02) erstreckt.

3. Mikrorobotische Einheit (01) nach Anspruch 1 oder 2, **dadurch gekennzeichnet, dass** axial vor dem vorderen Spreizmodul (02) ein kegelstumpfförmiger Dorn (07) angebracht ist, der eine zentrale Durchtrittsöffnung (08) aufweist.

4. Mikrorobotische Einheit (01) nach einem der Ansprüche 1 bis 3, **dadurch gekennzeichnet, dass** sie weiterhin ein Schlauchsystem (06) umfasst, welches an den Versorgungsanschluss (05) angekoppelt ist.

5. Mikrorobotische Einheit (01) nach einem der Ansprüche 1 bis 4, **dadurch gekennzeichnet, dass** sie ein oder mehrere weitere Module besitzt, die axial hinter dem hinteren Spreizmodul (03) angeordnet sind, wobei die weiteren Module insbesondere Vorratsbehälter, Batterieeinheiten und/oder Datenverarbeitungskomponenten umfassen.

6. Mikrorobotische Einheit (01) nach einem der Ansprüche 1 bis 5, **dadurch gekennzeichnet, dass** sie einen oder mehrere Sensoren umfasst, welche Umgebungsparameter und/oder Zustandsparameter der Einheit erfassen.

7. Mikrorobotische Einheit (01) nach einem der Ansprüche 1 bis 6, **dadurch gekennzeichnet, dass** die Faltenbalgstruktur (22) von einer äußeren Schraubenfeder oder einer Nitinol-Struktur (24) umgeben ist.

8. Mikrorobotische Einheit (01) nach einem der Ansprüche 1 bis 7, **dadurch gekennzeichnet, dass** im Inneren der Faltenbalgstruktur (22) eine innere Schraubenfeder (23) mit elastischer Hülle angeordnet ist.

9. Mikrorobotische Einheit (01) nach einem der Ansprüche 1 bis 8, **dadurch gekennzeichnet, dass** seitlich an der Faltenbalgstruktur (22) Lenkelemente (50) verlaufen, die zur Lenkung der Vorschubrichtung der Einheit aktivierbar sind.

10. Mikrorobotische Einheit (01) nach Anspruch 2, **dadurch gekennzeichnet, dass** am vorderen Ende des Zentralkanals (06a) ein Klappenmechanismus (60) zum wahlweisen Verschließen und Öffnen des Zentralkanals (06a) angeordnet ist.

11. Mikrorobotische Einheit (01) nach Anspruch 2 oder 10, **dadurch gekennzeichnet, dass** im Zentralkanal (06a) ein Arretierungsmechanismus (61) angeordnet ist, mit welchem im Zentralkanal geführte Instrumente festlegbar sind.

12. Mikrorobotische Einheit (01) nach einem der Ansprüche 1 bis 11, **dadurch gekennzeichnet, dass** die Faltenbalgstruktur (22) in ihrer Außenwandung mehrere aneinandergereihte Kniehebelelemente aufweist.

13. Mikrorobotische Einheit (01) nach einem der Ansprüche 1 bis 11, **dadurch gekennzeichnet, dass** die Faltenbalgstruktur (22) auf oder in ihrer Außenwandung eine Struktur aus einer Legierung mit Formgedächtnis oder aus Kunststoff aufweist.

## Claims

1. Microrobotic unit (01) for propulsion movement and positioning in organic cavities, comprising:
- a front spreadable module (02) and a rear spreadable module (03), wherein each spreadable module (02, 03) having at least one outer balloon envelope (20) the volume of which expands at least partially when the supply medium is supplied, wherein a surface structure (21) being provided on the outside of the balloon envelope (20) which, when expanded, anchors itself in the surrounding tissue;
- a pushing module (04) which is located between the two spreadable modules (02, 03) and connected to them, wherein the pushing module (04) has a bellows structure (22) which causes an axial expansion of the pushing module when the supply medium is supplied and an axial contraction of the pushing module when the supply medium is removed, wherein the bellows structure (22) has an outer, axially extending support structure in order to prevent or limit a change in its dimensions in the radial direction;
- a supply connection (05) which is axially connected to the rear spreadable module (03) and is used to supply at least one supply medium;
**characterized in that**
- the surface structure (21) of the balloon envelope (20) has a plurality of domes (71) which can be expanded in the radial direction of the balloon envelope and on which bristles (72) are formed which can also be expanded in the radial direction of the balloon envelope;
- the axially extending support structure of the bellows structure (22) is shaped like a plurality of toggle lever structures arranged in a row.

2. Microrobotic unit (01) according to claim 1, **characterized in that** a central channel (06b) extends through the rear spreadable module (03) and the pushing module (04) to the front spreadable module (02).

3. Microrobotic unit (01) according to claim 1 or 2, **characterized in that** a frustoconical mandrel (07) having a central passage opening (08) is mounted axially in front of the front spreadable module (02).

4. Microrobotic unit (01) according to one of claims 1 to 3, **characterized in that** it further comprises a hose system (06) which is coupled to the supply connection (05).

5. Microrobotic unit (01) according to one of claims 1 to 4, **characterized in that** it has one or more further modules which are arranged axially behind the rear spreadable module (03), wherein the further modules in particular comprise storage containers, battery units and/or data processing components.

6. Microrobotic unit (01) according to one of claims 1 to 5, **characterized in that** it comprises one or more sensors which detect environmental parameters and/or state parameters of the unit.

7. Microrobotic unit (01) according to one of claims 1 to 6, **characterized in that** the bellows structure (22) is surrounded by an outer coil spring or a Nitinol structure (24).

8. Microrobotic unit (01) according to one of claims 1 to 7, **characterized in that** an inner coil spring (23) with an elastic sheath is arranged in the interior of the bellows structure (22).

9. Microrobotic unit (01) according to one of claims 1 to 8, **characterized in that** steering elements (50) run laterally on the bellows structure (22) and can be activated to direct the feed direction of the unit.

10. Microrobotic unit (01) according to claim 2, **characterized in that** a flap mechanism (60) for selectively closing and opening the central channel (06a) is arranged at the front end of the central channel (06a).

11. Microrobotic unit (01) according to claim 2 or 10, **characterized in that** in the central channel (06a) there is arranged a locking mechanism (61), with which instruments guided in the central channel can be fixed.

12. Microrobotic unit (01) according to one of claims 1 to 11, **characterized in that** the bellows structure (22) has a plurality of toggle lever elements arranged in a row in its outer wall.

13. Microrobotic unit (01) according to one of claims 1 to 11, **characterized in that** the bellows structure (22) has a structure made of a shape memory alloy or of plastic on or in its outer wall.

## Revendications

1. Unité microrobotique (01), destinée à se déplacer et se positionner dans des cavités organiques, comprenant:
- un module d'expansion (02) antérieur et un module d'expansion (03) postérieur, chaque module d'expansion (02, 03) détenant au moins une enveloppe ballon (20) extérieure, dont la dilatation s'élargit au moins partiellement lors de l'apport du fluide d'alimentation, sur la face extérieure de l'enveloppe ballon (20) étant prévue une structuration superficielle (21), qui à l'état dilaté, provoque un ancrage dans le tissu environnant;
- un module de poussée (04), lequel est placé entre les deux modules d'expansion (02, 03) et est assemblé avec ceux-ci, le module de poussée (04) présentant une structure en soufflet (22), qui lors de l'apport du fluide d'alimentation provoque une dilatation axiale et lors de l'évacuation du fluide d'alimentation, provoque un raccourcissement axial du module de poussée, la structure en soufflet (22) présentant une structure d'appui extérieure, s'étendant axialement, pour empêcher ou pour limiter une modification de sa dilatation dans la direction radiale;
- un raccord d'alimentation (05), lequel se raccorde axialement sur le module d'expansion (03) postérieur et sert à l'apport d'au moins un fluide d'alimentation;
**caractérisée en ce que**
- la structuration superficielle (21) de l'enveloppe ballon (20) détient plusieurs coupoles (71) dilatables dans la direction radiale de l'enveloppe ballon, sur lesquelles sont conçus des poils (72), qui sont également dilatables dans la direction radiale de l'enveloppe ballon;
- la structure d'appui de la structure en soufflet (22) qui s'étend axialement étant façonnée à la manière de plusieurs structures à genouillère juxtaposées.

2. Unité microrobotique (01) selon la revendication 1, **caractérisée en ce qu'**un conduit central (06b) s'étend à travers le module d'expansion (03) postérieur et le module de poussée (04) s'étend jusqu'au module d'expansion (02) antérieur.

3. Unité microrobotique (01) selon la revendication 1 ou 2, **caractérisée en ce qu'**axialement à l'avant du module d'expansion (02) antérieur est monté un mandrin (07) de forme tronconique, qui présente un orifice de passage (08) central.

4. Unité microrobotique (01) selon l'une quelconque des revendications 1 à 3, **caractérisée en ce qu'**elle comprend par ailleurs un système de flexibles (06), lequel est accouplé sur le raccord d'alimentation (05).

5. Unité microrobotique (01) selon l'une quelconque des revendications 1 à 4, **caractérisée en ce qu'**elle détient un ou plusieurs modules supplémentaires, qui sont placés axialement derrière le module d'expansion (03) postérieur, les modules supplémentaires comprenant notamment des récipients de réserve, des unités de batterie et / ou des composants de traitement de données.

6. Unité microrobotique (01) selon l'une quelconque des revendications 1 à 5, **caractérisée en ce qu'**elle comprend un ou plusieurs capteurs, lesquels détectent des paramètres ambiants et / ou des paramètres d'état de l'unité.

7. Unité microrobotique (01) selon l'une quelconque des revendications 1 à 6, **caractérisée en ce que** la structure en soufflet (22) est entourée d'un ressort hélicoïdal extérieur ou d'une structure en Nitinol (24).

8. Unité microrobotique (01) selon l'une quelconque des revendications 1 à 7, **caractérisée en ce qu'**à l'intérieur de la structure en soufflet (22) est placée un ressort hélicoïdal (23) intérieur, doté d'une enveloppe élastique.

9. Unité microrobotique (01) selon l'une quelconque des revendications 1 à 8, **caractérisée en ce que** latéralement sur la structure en soufflet (22) s'écoulent des éléments de direction (50), qui sont activables pour diriger la direction d'avance de l'unité.

10. Unité microrobotique (01) selon la revendication 2, **caractérisée en ce que** sur l'extrémité antérieure du conduit central (06a) est placé un mécanisme à clapet (60), destiné à fermer et ouvrir sélectivement le conduit central (06a).

11. Unité microrobotique (01) selon la revendication 2 ou 10, **caractérisée en ce que** dans le conduit central (06a) est placé un mécanisme de blocage (61), à l'aide duquel des instruments guidés dans le conduit central sont immobilisables.

12. Unité microrobotique (01) selon l'une quelconque des revendications 1 à 11, **caractérisée en ce que** la structure en soufflet (22) présente dans sa paroi extérieure plusieurs éléments à genouillère juxtaposés.

13. Unité microrobotique (01) selon l'une quelconque des revendications 1 à 11, **caractérisée en ce que** la structure en soufflet (22) présente sur ou dans sa paroi extérieure une structure en un alliage à mémoire de forme ou en une matière plastique.
